# EUROPÄISCHE PATENTSCHRIFT

(11) **EP 3 897 469 B1**
(45) Veröffentlichungstag und Bekanntmachung des Hinweises auf die Patenterteilung: **25.01.2023**
(21) Anmeldenummer: 19820732.6
(22) Anmeldetag: 10.12.2019
(51) Int. Cl.: A61F 2/68, A61F 5/01, A61F 2/74

(54) **ORTHOPÄDIETECHNISCHE GELENKEINRICHTUNG**
ORTHOPEDIC JOINT DEVICE
DISPOSITIF D'ARTICULATION ORTHOPÉDIQUE

(30) Priorität: 20.12.2018 DE 102018133063
(43) Veröffentlichungstag der Anmeldung: 27.10.2021
(73) Patentinhaber: Ottobock SE & Co. KGaA, 37115 Duderstadt (DE)
(72) Erfinder: PUSCH, Martin, 37115 Duderstadt (DE)
(74) Vertreter: Gramm, Lins & Partner Patent- und Rechtsanwälte PartGmbB
(86) Internationale Anmeldenummer: PCT/EP2019/084499
(87) Internationale Veröffentlichungsnummer: WO 2020/126697

(56) Entgegenhaltungen:
- EP-A1- 3 137 020
- GB-A- 2 367 753

## Beschreibung

Die Erfindung betrifft eine orthopädietechnische Gelenkeinrichtung mit einem Oberteil und einem gelenkig um eine Schwenkachse an dem Oberteil befestigten Unterteil mit einer zwischen dem Oberteil und Unterteil angeordneten, flexionsmomentgesteuerten Halteeinrichtung, die eine Flexion sperrt und bei Überschreitung eines vorbestimmten Flexionsmomentes die Flexion freigibt.

Die orthopädietechnische Gelenkeinrichtung ist insbesondere als ein künstliches Prothesengelenk, Gelenk in Exoskeletten oder Orthesengelenk ausgebildet, insbesondere als ein künstliches Kniegelenk oder Ellenbogengelenk, ist jedoch nicht auf solche Ausgestaltungen beschränkt, sondern kann auch in anderen Bereichen eingesetzt werden. Bei einer Ausgestaltung als künstliches Kniegelenk ist mit einer solchen orthopädietechnischen Gelenkeinrichtung das Stehen und ggf. Gehen mit einem gesperrten Gelenk möglich, nach der Freigabe in Flexionsrichtung ist eine Verschwenkbarkeit des Oberteils relativ zum Unterteil gegeben. Die Gelenkeinrichtung kann jedoch auch als ein sogenanntes dynamisches Gelenk eingesetzt werden, mit dem spastischen Kontrakturen entgegengewirkt werden können oder über das mittels Dehnübungen die Kontrakturen therapiert werden können.

Sperrgelenke, insbesondere sogenannte Sperrkniegelenke, werden häufig bei wenig mobilen Patienten eingesetzt, bei denen eine eingeschränkte Mobilität beispielsweise durch Erkrankungen der unteren Extremität begründet sein kann. Aufgrund der eingeschränkten Mobilität besteht hier ein Bedarf lediglich an Kniegelenken, die ein einschränkungsfreies Sitzen ermöglichen und bei Mobilitätsaktionen einen Größtmaß an Sicherheit bereitstellen. Dazu sehen solche Kniegelenke in der Regel nur zwei Zustände vor, nämlich eine verriegelte Strickstellung und eine Entriegelungsstellung, damit das Kniegelenk frei bewegbar ist. Eine Anpassung der Dämpfung in eine Schwungphase oder Standphase ist häufig nicht vorgesehen. Ein solches künstliches Kniegelenk in Gestalt eines Prothesenkniegelenkes ist in der DE 20 2006 007 641 U1 beschrieben. Zur Entriegelung eines solchen Prothesenkniegelenkes ist es notwendig, einen Entriegelungsmechanismus per Hand zu betätigen.

Die DE 10 2008 024 747 A1 betrifft eine orthopädische Einrichtung mit einem Gelenk, das ein Oberteil schwenkbar mit einem Unterteil verbindet. An dem Oberteil sind Anschlussmittel zur Befestigung an einer Gliedmaße angeordnet. Eine Verriegelungseinrichtung verhindert eine Beugebewegung des Oberteils relativ zu dem Unterteil, wobei die Verriegelungseinrichtung aktiv durch den Nutzer der orthopädischen Einrichtung betätigbar ist. Eine Steuereinrichtung ist der Verriegelungseinrichtung zugeordnet, die mit zumindest einem an oder in der orthopädietechnischen Einrichtung angeordneten Sensor verbunden ist, so dass in Abhängigkeit von dem Sensorsignal die Verriegelungseinrichtung automatisch entriegelt oder gesperrt wird. Die Entriegelung oder Verriegelung kann auch durch ein Zeitschaltglied verzögert werden, sodass diese erst kurz nach dem eigentlichen Sensorsignal erfolgt.

Eine orthopädietechnische Gelenkeinrichtung gemäß dem Oberbegriff ist beispielsweise in der DE 10 2015 116 149 A1 beschrieben.

Problematisch kann hierbei sein, dass das Flexionsmoment, das notwendig ist, um die Halteeinrichtung zu deaktivieren, so hoch eingestellt ist, dass Nutzer der orthopädietechnischen Gelenkeinrichtung ein solches Moment nicht aufbringen können. Eine Verringerung des Freigabemomentes kann gegebenenfalls aus Sicherheitsüberlegungen keine Option sein, da ein Einbeugen während des Gehens nicht erwünscht sein kann.

Die GB 2 367 753 A betrifft eine Prothese der unteren Extremität, die eine Flexions-Steuervorrichtung aus einer Kombination aus Pneumatik- und Hydraulikdämpfer gebildet. Hierzu sind eine Pneumatikkammer und eine Hydraulikkammer ausgebildet. In beiden Kammern ist jeweils ein Kolben angeordnet, wobei beide Kolben an derselben Kolbenstange festgelegt sind. Der Hydraulikkammer sind zwei Bypässe zugeordnet, in denen jeweils ein Drehventil angeordnet ist.

Die EP 3 137 020 A1 betrifft ein Prothesen-Knöchelgelenk, bei dem ein Verbindungselement mit Gelenkfunktion ein Fußteil mit einem Unterschenkel-Anschlussteil verbindet. Eine Freigabevorrichtung ist in eine Arretierstellung, in der das Fußteil bezüglich einer Dorsalextensionsbewegung mit dem Unterschenkelanschlussteil drehstarr verbunden ist und eine Freigabestellung, in der eine Verdrehung möglich ist, bringbar. Die Freigabevorrichtung ist zum passiven Umschalten von Arretierstellung in die Freigabestellung, wenn eine zu einem Knöcheldrehmoment im Verbindungselement korrelierende Größe oberhalb eines voreingestellten Schwellwertes liegt, ausgebildet.

Aufgabe der vorliegenden Erfindung ist es daher, eine orthopädietechnische Gelenkeinrichtung bereitzustellen, die bei gleicher Sicherheit eine breite Anwendbarkeit auch für Patienten bereitstellt, die nur ein geringes Flexionsmoment aufbringen können.

Erfindungsgemäß wird diese Aufgabe durch eine orthopädietechnische Gelenkeinrichtung mit den Merkmalen des Hauptanspruches erreicht. Vorteilhafte Ausgestaltungen und Weiterbildungen der Erfindung sind in den Unteransprüchen, der Beschreibung sowie den Figuren offenbart.

Die orthopädietechnische Gelenkeinrichtung mit einem Oberteil und einem gelenkig um eine Schwenkachse an dem Oberteil befestigten Unterteil mit einer zwischen dem Oberteil und dem Unterteil angeordneten, flexionsmomentgesteuerten Halteeinrichtung, die eine Flexion sperrt und bei Überschreiten eines vorbestimmten Flexionsmomentes die Flexion freigibt, sieht vor, dass der Halteeinrichtung ein Stellelement zugeordnet ist, das mit einem Verzögerungselement gekoppelt ist, das eine zeitlich verzögerte Aktivierung des Stellelementes bewirkt. Mit dem Stellelement und der Kombination des Stellelementes mit einem Verzögerungselement ist es möglich, dass ein geringeres Flexionsmoment als Freigabemoment aufgebracht werden muss, um eine Freigabe der Halteeinrichtung zu bewirken. Das vergleichsweise geringere Moment gegenüber herkömmlichen Gelenkeinrichtungen muss jedoch über einen längeren Zeitraum aufgebracht werden, so dass das Integral des Momentes über der Zeit zumindest gleich, wenn nicht sogar größer als das Integral ohne ein Verzögerungselement ist. Dadurch ist es möglich eine Freigabe auch dann zu bewirken, wenn kein hohes Momentanmoment aufgebracht werden kann, das ein schlagartiges Lösen der Halteeinrichtung bewirkt. Die Flexionssperre kann somit aufgehoben werden, ohne dass die Sicherheit bei einem Gehen oder Stehen beeinträchtigt wird, da das Sperrmoment hoch eingestellt bleiben kann. Ein vergleichsweise geringes Flexionsmoment muss über einen vergleichsweise langen Zeitraum aufgebracht werden, bevor die Halteeinrichtung freigeschaltet wird, um die Flexion freizugeben, was beispielsweise durch eine dauerhafte Belastung in Flexionsrichtung bei dem Wunsch, sich hinzusetzen erfüllt ist. Je nach Auslegung des Verzögerungselementes muss über einen angepassten und anpassbaren Zeitraum das erforderliche Minimalflexionsmoment aufgebracht werden, bevor die Halteeinrichtung freigegeben wird. Über das Verzögerungselement lässt sich die Zeitdauer für das Aufbringen des Flexionsmomentes einstellen und an den jeweiligen Nutzer der Gelenkeinrichtung anpassen. Somit erreicht man eine automatische Entsperrung ohne eine manuelle Entriegelung oder durch eine aufwendige Sensorik. Das Verzögerungselement kann insbesondere als ein mechanisches und/oder hydraulisches Verzögerungselement ausgebildet sein.

Die Halteeinrichtung kann als eine mechanische Bremse oder als pneumatisches oder hydraulisches Dämpfersystem oder als eine pneumatische oder hydraulische Dämpfereinrichtung ausgebildet sein. Die mechanische Bremse kann beispielsweise als Klemmfaust oder Klemmeinrichtung ausgebildet sein, die eine Achse der Gelenkeinrichtung aufnimmt und die Klemmung reduziert und eine Flexionsbewegung freigibt, wenn über eine ausreichende Zeitdauer ein ausreichend hohes Flexionsmoment ausgeübt wird. Die Klemmeinrichtung kann integraler Bestandteil eines Gelenkkörpers sein, in oder an dem das Oberteil und/oder das Unterteil gelagert sind. Sie kann als Schlingfederbremse, Backenbremse oder als andere mechanische Bremseinrichtung ausgebildet sein. Alternativ dazu kann eine fluidbetätigte Dämpfereinrichtung, also eine pneumatische oder hydraulische Dämpfereinrichtung, die Halteeinrichtung ausbilden, bei der eine Strömungsbewegung gesperrt wird, um die Flexion zu sperren. Dazu ist beispielsweise ein Ventil oder eine andere Sperreinrichtung vorgesehen, die in einer Fluidleitung angeordnet ist, durch die bei einer ungehinderten Flexionsbewegung das Fluid strömen würde. Beispielsweise kann in einer Hydraulikleitung von einer ersten Kammer in eine zweite Kammer eines Zylinders mit einem beweglichen Kolben oder von einer ersten Kammer in einen Ausgleichsbehälter ein entsprechendes Stellventil oder eine Sperreinrichtung angeordnet sein, die mit einem Verzögerungselement gekoppelt ist. Das Stellelement öffnet erst nach einem vorbestimmten Zeitraum der Aufbringung eines Flexionsmomentes das Ventil oder die andere Sperreinrichtung, vorzugsweise langsam, um eine schlagartige Freigabe und damit ein plötzliches Einbeugen der Gelenkeinrichtung unter Last zu vermeiden.

Die Dämpfereinrichtung kann einen Zylinder aufweisen, in dem ein beweglicher Kolben gelagert ist. Der Zylinder kann sowohl als Rotationszylinder als auch als Linearzylinder ausgebildet sein, in dem der Kolben entweder eine Schwenkbewegung oder eine lineare reziproke Bewegung entlang der Zylinderwandung an einer Kolbenstange oder einem Pleuel ausführt. Der Kolben unterteilt den Zylinder in zumindest zwei Kammern, so dass bei einer Verlagerung des Kolbens und damit einhergehender Verringerung eines Kammervolumens einer ersten Kammer Fluid von der ersten Kammer durch eine Leitung aus der Kammer strömt, beispielsweise in die zumindest eine weitere Kammer oder in einen Ausgleichsbehälter. Innerhalb der Leitung oder dem Strömungsweg des Fluides ist eine Stellventil angeordnet, dem wiederum das Stellelement zugeordnet ist, um den Strömungswiderstand innerhalb der Leitung oder des Strömungsweges einzustellen, also um bei Aufbringung eines ausreichend hohen Flexionsmomentes über einen ausreichend langen Zeitraum, die einstellbar sind, die Sperrung aufzuheben und damit den Strömungswiderstand von quasi unendlich zu reduzieren.

Das Stellelement kann als ein pneumatischer oder hydraulischer Aktuator ausgebildet sein, der mit zumindest einer Kammer der Dämpfereinrichtung in strömungstechnischer Bindung steht. Der Aktuator ist beispielsweise als Schieber, Kolben oder ähnliches druckbetätigtes Element ausgebildet, das über den aufgebrachten Druck innerhalb der Leitung aufgrund des aufgebrachten Flexionsmomentes eine Verlagerung des Stellelementes und damit eine Verringerung oder Veränderung des Strömungswiderstandes bewirkt.

In einer Weiterbildung der Erfindung ist es vorgesehen, dass das Stellelement in entgegengesetzten Richtungen antreibbar ausgebildet ist, so dass beispielsweise bei einem aufgebrachten Extensionselement das Stellelement eine umgekehrte Bewegung ausführt, also insbesondere den Strömungswiderstand erhöht und die Fluidleitung schließt oder die mechanische Bremse aktuiert und eine Klemmung oder anderweitige Sperrung einer Flexionsbewegung bewirkt. Die Sperrung kann bei einer mechanischen Bremse auch durch eine Formschlusseinrichtung oder ein Formschlusselement ausgebildet werden, das in Eingriff oder außer Eingriff mit einem korrespondierenden Formschlusselement gebracht wird.

Bei einer Ausgestaltung der Halteeinrichtung mit einer Dämpfereinrichtung kann eine Rücklaufleitung mit einem Rückschlagventil vorgesehen sein, die mit dem Stellelement gekoppelt ist, um eine reziproke Bewegung des Stellelementes bei einem aufgebrachten Extensionsmoment zu bewirken.

Die Fluidleitung aus der ersten Kammer kann mit der anderen oder zweiten Kammer oder einem Ausgleichsvolumen verbunden sein, so dass bei Freigabe der Leitung Fluid aus der ersten Kammer herausströmen und eine Flexionsbewegung bevorzugt kontrolliert stattfinden kann.

Das Stellelement kann als Schieber, Hebel oder Drehgesperre ausgebildet sein, ebenfalls kann das Stellelement eine Hebelübersetzung, ein Getriebe oder ein Rückstellelement aufweisen, um eine Kraftübersetzung vornehmen zu können und darüber hinaus eine Umkehrbewegung durchzuführen, damit nach der gewünschten Flexionsbewegung oder nach einer gewissen Zeit die Sperrung der Flexionsbewegung wieder eingestellt oder aktiviert wird.

Das Verzögerungselement kann als ein Ventil in der Fluidleitung, als ein gedämpft gelagerter Schieber oder gedämpftes Bewegungsgewinde ausgebildet sein, dessen Verzögerungswert und damit der Zeitraum, innerhalb dessen ein Mindestmaß an Flexionsmoment aufgebracht werden muss, eingestellt werden kann.

Das Stellelement kann mit einem Kraftspeicher gegen eine Widerstandsverringerung gekoppelt sein, also beispielsweise mit einer Feder oder einem komprimierbaren Fluid gekoppelt sein, das das Stellelement grundsätzlich belastet. Dadurch wird sichergestellt, dass grundsätzlich eine Flexionsbewegung gesperrt wird und nach Wegfall der Stellkraft, mit der das Stellelement aus einer Sperrposition in eine Freigabeposition gebracht wird, die Sperrposition wieder eingenommen wird. Bevorzugt ist der Kraftspeicher, der dem Stellelement zugeordnet ist, einstellbar ausgebildet, um dadurch das notwendige, aufzubringende Flexionsmoment einstellen zu können.

Das Stellelement ist bevorzugt druckgesteuert und zeitverzögert aktuierbar ausgebildet, da über eine Drucksteuerung aufzubringende Stellkräfte leicht einstellbar und über entsprechende Leitungen leicht an dem jeweils benötigten Ort führbar sind. Ebenfalls können Druckübersetzungen durch Kolben-Zylinder-Anordnungen platzsparend ausgeführt werden. Es kann auch eine Kopplung von hydraulischer oder pneumatischer Aktuierung des Stellelementes und einer mechanischen Bremse erfolgen. Bei einer Halteeinrichtung mit einer Dämpfereinrichtung kann der hydraulische oder pneumatische Druck der Dämpfereinrichtung gleichzeitig zum Aktuieren des Stellelementes genutzt werden.

Die orthopädietechnische Gelenkeinrichtung ist als Orthesen- oder Prothesenkniegelenk oder Orthesen- oder Prothesenellenbogengelenk oder als Orthesen- oder Prothesenhandgelenk oder als entsprechendes Gelenk eines Exoskelettes ausgebildet.

Eine Dämpfereinrichtung, wie sie oben beschrieben worden ist, kann auch außerhalb von orthopädietechnischen Gelenkeinrichtungen im Gebiet der Orthopädietechnik vorteilhaft eingesetzt werden, beispielsweise zur zeitgesteuerten und kontrollierten Freigabe von Linearbewegungen.

Nachfolgend wird die Erfindung anhand von Ausführungsbeispielen näher erläutert. Es zeigen:
- Figur 1: eine orthopädietechnische Gelenkeinrichtung mit einer Dämpfereinrichtung;
- Figur 2: eine orthopädietechnische Gelenkeinrichtung mit einer mechanischen Bremse; sowie
- Figur 3: eine Variante der Figur 1.

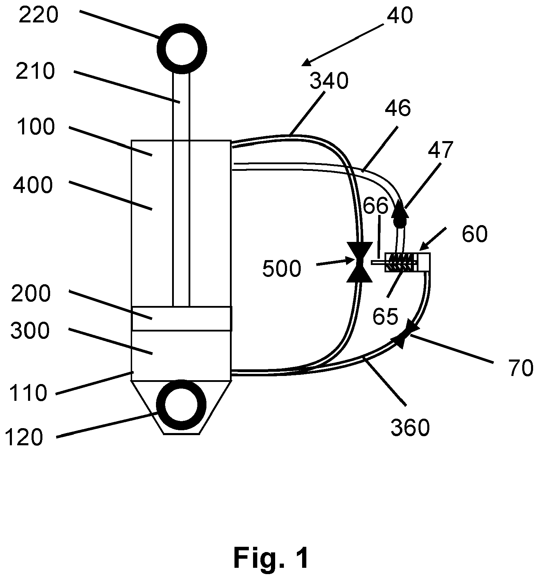

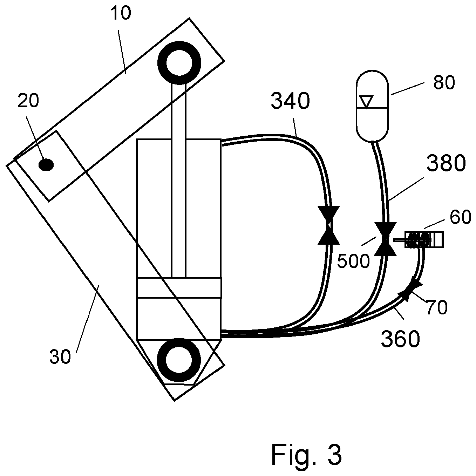

In der Figur 1 ist in einer schematischen Darstellung eine Halteeinrichtung 40 in Gestalt einer hydraulischen Dämpfereinrichtung dargestellt. Die Halteeinrichtung 40 ist zwischen einem nicht dargestellten Oberteil 10 und einem gelenkig um eine Schwenkachse 20 in dem Oberteil 10 befestigten Unterteil 30 angeordnet. Aus Gründen der Übersichtlichkeit sind das Oberteil 10 und das Unterteil 30 in der Figur 3 eingezeichnet. Die Halteeinrichtung 40 in Gestalt einer fluidischen Dämpfereinrichtung, insbesondere einer hydraulischen Dämpfereinrichtung, weist einen Zylinder 100 mit einem Zylindergehäuse 110 auf, in dem ein Kolben 200 längsverschieblich gelagert ist. Der Kolben 200 unterteilt den Zylinder 100 in zwei Kammern 300, 400, die strömungstechnisch über eine Leitung 340 miteinander verbunden sind. Der Kolben 200 ist an einer Kolbenstange 210 geführt, die aus dem Gehäuse 110 herausragt. An dem dem Kolben 200 abgewandten Ende der Kolbenstange 210 ist eine Lagerung oder Achsaufnahme 220 angeordnet oder ausgebildet. An dem der Lagerung oder der Achsaufnahme 220 gegenüberliegenden Ende der Dämpfereinrichtung ist an dem Gehäuse 110 eine korrespondierende Lagerung oder Achsaufnahme 120 angeordnet oder ausgebildet. Ein Fluidreservoir kann vorgesehen sein, um die unterschiedlichen Volumenänderungen in den beiden Kammern 300, 400 aufgrund der eintauchenden oder ausfahrenden Kolbenstange 210 auszugleichen. Sofern in der unteren ersten Kammer 300 ebenfalls eine Kolbenstange angeordnet ist, kann auf ein Reservoir verzichtet werden.

In dem dargestellten Ausführungsbeispiel ist die Strömungsleitung 340 von der ersten Kammer 300 in die zweite Kammer 400 mit einem Stellventil 500 versehen, das hinsichtlich der Durchlassmenge und damit hinsichtlich des Strömungswiderstandes innerhalb der Leitung 340 einstellbar ist. Durch die Veränderung des Strömungswiderstandes innerhalb der Leitung 340 kann der Widerstand gegen eine Abwärtsbewegung oder Aufwärtsbewegung des Kolbens 200 und damit entgegen einer Volumenverringerung oder Vergrößerung in einer der beiden Kammern 300, 400 eingestellt werden, beispielsweise um einen Flexionswiderstand oder einen Extensionswiderstand einzustellen. Die Widerstände können je nach Bewegungsrichtung unterschiedlich eingestellt werden, beispielsweise über ein Mehrwegeventil mit Rückschlagventil, die druckgesteuert oder sensorgesteuert einen Fluidstrom in die eine oder andere Strömungsrichtung sperren oder freigeben.

Dem Stellventil 500 zugeordnet ist ein Stellelement 60, das druckgesteuert betrieben wird. Wird der Kolben 200 durch eine Flexion der Gelenkeinrichtung nach unten gedrückt, wird die erste Kammer 300 verkleinert, so dass das Fluid, beispielsweise Hydrauliköl, durch die Leitung 340 aus der ersten Kammer 300 ausströmt. Das Stellventil 500 ist dabei geschlossen. Von der Leitung 340 zweigt eine Druckleitung 360 ab, die zu dem Stellelement 60 führt. Das Stellelement 60 weist einen Kolben in einem Zylinder mit einem federbelasteten Schieber oder Aktuator 66 auf. Der Kolben innerhalb des Stellelementes 60 drückt gegen die Feder 65 als Kraftspeicher und verlagert den Schieber 66 in Richtung auf das Stellventil 500. Wird mit einer ausreichenden Kraft auf den Kolben 200 gedrückt, gibt sich eine entsprechend hohe Druckkraft auf den Kolben innerhalb des Stellelementes 60, so dass je nach Einstellung des Kraftspeichers 65 oder der Vorspannung der Feder ab einem bestimmten Flexionsmoment und der einhergehenden Druckkraft auf den Kolben eine entsprechende Verlagerung des Stempels 66 erfolgt. Über den Stempel 66 wird das Stellventil 500 geöffnet und der Strömungswiderstand in der Leitung 340 verringert, so dass nach einer anfänglichen Sperrung der Flexionsbewegung ein Volumenausgleich von der ersten Kammer 300 in die zweite Kammer 400 erfolgen kann, so dass eine Flexion erfolgt.

Um eine zeitliche Verzögerung der Aktivierung des Stellelementes 60 und damit der Öffnung der Leitung 340 durch die Verstellung des Stellventils 500 zu erreichen, ist in der Druckleitung 360, die zu dem Stellelement 60 führt, ein Ventil 70 oder eine Drossel angeordnet, über die der Strömungswiderstand in der Leitung 360 und damit der Druckaufbau in dem Stellelement 60 verzögert wird. Je weniger Fluid aus der unteren Kammer 300 durch das Ventil 70 oder die Drossel 70 in den Zylinder des Stellelementes 60 gelangen kann, um auf den dort angeordneten Kolben zu wirken, desto länger muss das Flexionsmoment aufgebracht werden, um den Schieber 66 dahingehend zu verlagern, dass das Stellventil 500 geöffnet wird.

Zur Rückstellung des Schiebers 66 oder zur Deaktivierung des Stellelementes 60 kann entweder alleine der Kraftspeicher 65 ausreichen, der den Kolben innerhalb des Stellelementes 60 in die Ausgangsposition zurückdrückt oder eine Rückströmleitung 64 aus der oberen Kammer 400 ist über ein Rückschlagventil 47 mit einer Kolbenkammer des Stellelementes 60 verbunden, so dass bei einer Extensionsbewegung aufgrund des hydraulischen Druckes eine Rückstellkraft ausgeübt wird. Ein Auslassventil oder eine andere Druckminderungseinrichtung innerhalb des Stellelementes 60 ist vorgesehen, um eine hydraulische Blockade zu vermeiden.

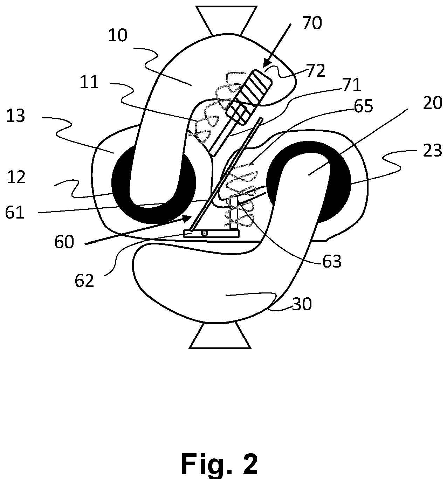

Eine Variante der Erfindung ist in der Figur 2 dargestellt, bei der die orthopädietechnische Gelenkeinrichtung als ein fremdes Kniegelenk mit dem Oberteil 10 und dem Unterteil 30 gezeigt ist. Ein Gelenkkörper 13 in Gestalt einer Bremsschelle nimmt das Oberteil 10 schwenkbar auf. Das Oberteil 10 ist in dem Gelenkkörper 13 mit einer Steuerachse 12 verschwenkbar gelagert. Analog dazu ist das Unterteil 30 mit einer blockierbaren Steuerachse 23 um die Schwenkachse 20 schwenkbar gelagert. Zusammen mit dem Gelenkkörper 13 verschwenkt das Oberteil 10 um die Schwenkachse 20, wenn ein ausreichend großes Flexionsmoment über eine ausreichend lange Zeit aufgebracht worden ist. Die Steuerachse 23 ist gleichzeitig eine Bremsachse, da der Gelenkkörper 13 diese unter Freilassung eines Spaltes fast vollständig umfänglich umgibt und klemmend hält. Wird über das Oberteil 10 ein ausreichendes Flexionsmoment aufgebracht, um eine Verlagerung um die Steuerachse 12 im Uhrzeigersinn zu bewirken, drückt das Oberteil eine mit einem Gewinde versehene Buchse 72 in Richtung auf den Gelenkkörper 13. Die Buchse 72 stützt sich über einen Stößel oder eine Stange 71 auf dem Gelenkkörper 13 ab. Zwischen dem Gelenkkörper 13 und dem Oberteil 10 ist zudem eine Feder 11 oder ein anderer Kraftspeicher angeordnet, der einer Verschwenkung im Uhrzeigersinn um die Steuerachse 12 entgegenwirkt. Der Stößel 71 ist zumindest einem korrespondierenden Gewinde versehen, so dass bei einem ausreichenden Flexionsmoment der Stößel 71 in die Buchse 72 hineingedreht wird. Alternativ kann eine hydraulische Dämpfung der Bewegung des Stößels 71 erfolgen. Das Gewinde innerhalb der Buchse 72 kann mit einer hochviskosen Beschichtung versehen sein, die zusätzlich die Einschraub- oder Eindrehbewegung dämpft, so dass sich der Stößel 71 nur langsam in die Buchse 72 hineindreht. Die Anordnung aus Feder 11 Buchse 72 und Stößel 71 stellt ein Verzögerungselement 70 dar, das eine Verschwenkung im Uhrzeigersinn um die Steuerachse 12 verlangsamt. Ist eine ausreichende Verschwenkung des Oberteils 10 erreicht, wird ein Stab 61 oder eine Stange 61 in Kontakt mit dem Oberteil 10 gebracht. Die Stange 61 ist an einem Kipphebel 62 angeordnet, so dass bei einem Kontakt des Oberteils 10 mit der Stange 61 eine Verschwenkung um eine Kippachse erfolgt, so dass das Stangenende des Kipphebels 62 nach unten verlagert wird. Dadurch wird ein zweiter Stößel 63 nach oben verschwenkt und drückt gegen eine Klemmfaust, die Teil des Gelenkkörpers 13 ist und die zweite Steuerachse 320 durch eine Zugfeder 65 klemmend hält und eine entsprechende Verdrehung des Gelenkkörpers 13 im Uhrzeigersinn um die Schwenkachse 20 verhindert. Wenn eine ausreichend große Kraft durch den zweiten Stößel 63 aufgebracht worden ist, um die Bremse zu lösen, folgt bei einem weiterhin aufgebrachten Flexionsmoment eine Verschwenkung des Oberteils 10 im Uhrzeigersinn um die Schwenkachse 20 relativ zu dem Unterteil.

Bei nachlassendem Flexionsmoment oder Aufbringung eines Extensionsmomentes erfolgt eine erneute Klemmung zur Fixierung der zweiten Steuerachse 23. Um eine Rückstellbewegung zu ermöglichen, kann ein Ratschenmechanismus vorgesehen sein, so dass eine Extensionsbewegung stets möglich ist. Innerhalb des Verzögerungselementes 70 kann ein Rückstellmechanismus oder eine Rückstellfeder ausgebildet sein, die bei nachlassendem Flexionsmoment oder aufgebrachten Extensionsmoment den Stößel 71 in seine Ausgangsposition bringt.

Figur 3 zeigt eine Variante der Figur 1 mit einem im Wesentlichen gleichen Aufbau, so dass nicht alle Bezugszeichen eingezeichnet sind. In der Verbindungsleitung 340 zwischen den beiden Kammern 300, 400 ist ein Ventil oder eine Drossel angeordnet. Das Stellventil 500, das mit dem Stellelement 60 und dem Verzögerungselement 70 gekoppelt ist, ist in einer Druckleitung 380 angeordnet, das zu einem Ausgleichsbehälter 80 führt. Wird das Stellventil 500 nach Aufbringen eines ausreichenden Flexionsmomentes über einen ausreichenden Zeitraum geöffnet, strömt Fluid von der unteren Kammer durch die Druckleitung 380 in den Ausgleichsbehälter 80 und bewirkt eine Flexionsbewegung zwischen dem Oberteil 10 und dem Unterteil 30.

## Patentansprüche

1. Orthopädietechnische Gelenkeinrichtung mit einem Oberteil (10) und einem gelenkig um eine Schwenkachse (20) an dem Oberteil (10) befestigten Unterteil (30) mit einer zwischen dem Oberteil (10) und dem Unterteil (30) angeordneten, flexionsmomentgesteuerten Halteeinrichtung (40), die eine Flexion sperrt und bei Überschreiten eines vorbestimmten Flexionsmomentes die Flexion freigibt, **dadurch gekennzeichnet, dass** der Halteeinrichtung (40) ein Stellelement (60) zugeordnet ist, das mit einem Verzögerungselement (70) gekoppelt ist, das eine zeitlich verzögerte Aktivierung des Stellelementes (60) bewirkt.

2. Orthopädietechnische Gelenkeinrichtung nach Anspruch 1, **dadurch gekennzeichnet, dass** das Verzögerungselement (70) als ein mechanisches und/oder hydraulisches Verzögerungselement (70) ausgebildet ist.

3. Orthopädietechnische Gelenkeinrichtung nach Anspruch 1 oder 2, **dadurch gekennzeichnet, dass** die Halteeinrichtung (40) als mechanische Bremse oder als pneumatische oder hydraulische Dämpfereinrichtung ausgebildet ist.

4. Orthopädietechnische Gelenkeinrichtung nach Anspruch 3, **dadurch gekennzeichnet, dass** die Dämpfereinrichtung einen Zylinder (100) aufweist, in dem ein beweglicher Kolben (200) gelagert ist, der Kolben (200) unterteilt den Zylinder (100) in zwei Kammern (300, 400), so dass bei einer Verlagerung des Kolbens (200) bei Verringerung eines Kammervolumens Fluid von einer ersten Kammer (300) durch eine Leitung (340) aus der ersten Kammer (300) strömt, wobei innerhalb der Leitung (340) ein Stellventil (500) angeordnet ist, dem das Stellelement (60) zugeordnet ist, um einen Strömungswiderstand einzustellen.

5. Orthopädietechnische Gelenkeinrichtung nach einem der voranstehenden Ansprüche, **dadurch gekennzeichnet, dass** das Stellelement (60) als pneumatischer oder hydraulischer Aktuator ausgebildet ist, der mit zumindest einer Kammer (300, 400) in strömungstechnischer Verbindung steht.

6. Orthopädietechnische Gelenkeinrichtung nach einem der voranstehenden Ansprüche, **dadurch gekennzeichnet, dass** das Stellelement (60) in entgegengesetzten Richtungen antreibbar ausgebildet ist.

7. Orthopädietechnische Gelenkeinrichtung nach einem der voranstehenden Ansprüche, **dadurch gekennzeichnet, dass** eine Rücklaufleitung (46) mit Rückschlagventil (47) mit dem Stellelement (60) gekoppelt ist.

8. Orthopädietechnische Gelenkeinrichtung nach einem der Ansprüche 4 bis 7, **dadurch gekennzeichnet, dass** die Leitung (340) mit der anderen Kammer (40) und/oder einem Ausgleichsvolumen (80) verbunden ist.

9. Orthopädietechnische Gelenkeinrichtung nach einem der voranstehenden Ansprüche, **dadurch gekennzeichnet, dass** das Stellelement (60) als Schieber, Hebel oder Drehgesperre ausgebildet ist.

10. Orthopädietechnische Gelenkeinrichtung nach einem der voranstehenden Ansprüche, **dadurch gekennzeichnet, dass** das Verzögerungselement (70) als Ventil in einer Fluidleitung (36), gedämpft gelagerter Schieber oder gedämpftes Bewegungsgewinde ausgebildet ist.

11. Orthopädietechnische Gelenkeinrichtung nach einem der voranstehenden Ansprüche, **dadurch gekennzeichnet, dass** das Verzögerungselement (70) einstellbar ausgebildet ist.

12. Orthopädietechnische Gelenkeinrichtung nach einem der voranstehenden Ansprüche, **dadurch gekennzeichnet, dass** das Stellelement (60) mit einem Kraftspeicher (65) gegen eine Widerstandsverringerung gekoppelt ist.

13. Orthopädietechnische Gelenkeinrichtung nach Anspruch 12, **dadurch gekennzeichnet, dass** der Kraftspeicher (65) einstellbar ausgebildet ist.

14. Orthopädietechnische Gelenkeinrichtung nach einem der voranstehenden Ansprüche, **dadurch gekennzeichnet, dass** das Stellelement (60) druckgesteuert und zeitverzögert aktuierbar ausgebildet ist.

15. Orthopädietechnische Gelenkeinrichtung nach einem der voranstehenden Ansprüche, **dadurch gekennzeichnet, dass** die orthopädietechnische Gelenkeinrichtung als Orthesen- oder Prothesenkniegelenk oder Orthesen- oder Prothesenellenbogengelenk oder als Orthesen- oder Prothesenhandgelenk ausgebildet ist.

## Claims

1. An orthopedic joint device having an upper part (10) and a lower part (30) which is fastened to the upper part (10) in an articulated manner about a pivot axis (20), with a flexion-moment-controlled retaining device (40) which is arranged between the upper part (10) and the lower part (30) and which blocks flexion and releases the flexion when a predetermined flexion moment is exceeded, **characterized in that** the retaining device (40) is assigned a control element (60) which is coupled to a delay element (70), which causes a time-delayed activation of the actuating element (60).

2. The orthopedic joint device as claimed in claim 1, **characterized in that** the delay element (70) is configured as a mechanical and/or hydraulic delay element (70).

3. The orthopedic joint device as claimed in claim 1 or 2, **characterized in that** the retaining device (40) is configured as a mechanical brake or as a pneumatic or hydraulic damper device.

4. The orthopedic joint device as claimed in claim 3, **characterized in that** the damper device has a cylinder (100) in which a movable piston (200) is mounted, the piston (200) divides the cylinder (100) into two chambers (300, 400) such that, when the piston (200) is shifted accompanied by a reduction in a chamber volume, fluid from a first chamber (300) flows out of the first chamber (300) through a line (340), wherein inside the line (340) there is a control valve (500) which is assigned the control element (60) in order to set a flow resistance.

5. The orthopedic joint device as claimed in one of the preceding claims, **characterized in that** the control element (60) is configured as a pneumatic or hydraulic actuator which is connected in terms of flow to at least one chamber (300, 400).

6. The orthopedic joint device as claimed in one of the preceding claims, **characterized in that** the control element (60) is configured to be drivable in opposite directions.

7. The orthopedic joint device as claimed in one of the preceding claims, **characterized in that** a return line (46) with a check valve (47) is coupled to the control element (60).

8. The orthopedic joint device as claimed in one of claims 4 to 7, **characterized in that** the line (340) is connected to the other chamber (40) and/or to a compensating volume (80).

9. The orthopedic joint device as claimed in one of the preceding claims, **characterized in that** the control element (60) is configured as a slide, lever or rotary locking mechanism.

10. The orthopedic joint device as claimed in one of the preceding claims, **characterized in that** the delay element (70) is configured as a valve in a fluid line (36), as a slide mounted in a damped manner, or as a damped motion thread.

11. The orthopedic joint device as claimed in one of the preceding claims, **characterized in that** the delay element (70) is configured to be adjustable.

12. The orthopedic joint device as claimed in one of the preceding claims, **characterized in that** the control element (60) is coupled to a force accumulator (65) to counter a reduction in resistance.

13. The orthopedic joint device as claimed in claim 12, **characterized in that** the force accumulator (65) is configured to be adjustable.

14. The orthopedic joint device as claimed in one of the preceding claims, **characterized in that** the control element (60) is configured to be actuable under pressure control and with a time delay.

15. The orthopedic joint device as claimed in one of the preceding claims, **characterized in that** the orthopedic joint device is configured as an orthotic or prosthetic knee joint or orthotic or prosthetic elbow joint or as an orthotic or prosthetic wrist.

## Revendications

1. Dispositif d'articulation orthopédique comportant une partie supérieure (10) et une partie inférieure (30) qui est fixée à la partie supérieure (10) de manière articulée autour d'un axe de pivotement (20) et qui comprend un dispositif de retenue (40) disposé entre la partie supérieure (10) et la partie inférieure (30) et commandé par un couple de flexion, lequel bloque une flexion et libère la flexion en cas de dépassement d'un couple de flexion prédéterminé,
**caractérisé en ce qu'**au dispositif de retenue (40) est associé un élément de réglage (60) qui est couplé à un élément de temporisation (70) assurant une activation temporisée de l'élément de réglage (60).

2. Dispositif d'articulation orthopédique selon la revendication 1, **caractérisé en ce que** l'élément de temporisation (70) est réalisé sous la forme d'un élément de temporisation mécanique et/ou hydraulique (70).

3. Dispositif d'articulation orthopédique selon la revendication 1 ou 2, **caractérisé en ce que** le dispositif de retenue (40) est réalisé sous la forme d'un frein mécanique ou d'un dispositif d'amortissement pneumatique ou hydraulique.

4. Dispositif d'articulation orthopédique selon la revendication 3, **caractérisé en ce que** le dispositif d'amortissement présente un vérin (100) dans lequel est logé un piston mobile (200),
le piston (200) subdivise le vérin (100) en deux chambres (300, 400), de sorte que, lors d'un déplacement du piston (200), en cas de réduction d'un volume de chambre, du fluide s'écoule d'une première chambre (300) à travers une conduite (340) menant hors de la première chambre (300), une vanne de réglage (500) étant disposée à l'intérieur de la conduite (340), à laquelle est associé l'élément de réglage (60), afin de régler une résistance à l'écoulement.

5. Dispositif d'articulation orthopédique selon l'une des revendications précédentes,
**caractérisé en ce que** l'élément de réglage (60) est réalisé sous la forme d'un actionneur pneumatique ou hydraulique qui est en communication fluidique avec au moins une chambre (300, 400).

6. Dispositif d'articulation orthopédique selon l'une des revendications précédentes,
**caractérisé en ce que** l'élément de réglage (60) est réalisé de manière à pouvoir être entraîné dans des directions opposées.

7. Dispositif d'articulation orthopédique selon l'une des revendications précédentes,
**caractérisé en ce qu'**une conduite de retour (46) pourvue d'un clapet anti-retour (47) est couplée à l'élément de réglage (60).

8. Dispositif d'articulation orthopédique selon l'une des revendications 4 à 7,
**caractérisé en ce que** la conduite (340) est reliée à l'autre chambre (40) et/ou à un volume de compensation (80).

9. Dispositif d'articulation orthopédique selon l'une des revendications précédentes,
**caractérisé en ce que** l'élément de réglage (60) est réalisé sous la forme d'un coulisseau, d'un levier ou d'un moyen de blocage en rotation.

10. Dispositif d'articulation orthopédique selon l'une des revendications précédentes,
**caractérisé en ce que** l'élément de temporisation (70) est réalisé sous la forme d'une vanne dans une conduite à fluide (36), d'un coulisseau monté de manière amortie ou d'un filetage de déplacement amorti.

11. Dispositif d'articulation orthopédique selon l'une des revendications précédentes,
**caractérisé en ce que** l'élément de temporisation (70) est réalisé de façon réglable.

12. Dispositif d'articulation orthopédique selon l'une des revendications précédentes,
**caractérisé en ce que** l'élément de réglage (60) est couplé à un accumulateur de force (65) à l'encontre d'une diminution de la résistance.

13. Dispositif d'articulation orthopédique selon la revendication 12, **caractérisé en ce que** l'accumulateur de force (65) est réalisé de façon réglable.

14. Dispositif d'articulation orthopédique selon l'une des revendications précédentes,
**caractérisé en ce que** l'élément de réglage (60) est commandé par la pression et peut être actionné de façon temporisée.

15. Dispositif d'articulation orthopédique selon l'une des revendications précédentes,
**caractérisé en ce que** le dispositif d'articulation orthopédique est réalisé sous la forme d'une orthèse ou prothèse de l'articulation du genou, ou d'une orthèse ou prothèse de l'articulation du coude, ou d'une orthèse ou de prothèse du poignet.
